# EUROPEAN PATENT SPECIFICATION

(11) **EP 2 331 183 B1**
(45) Date of publication and mention of the grant of the patent: **04.06.2014**
(21) Application number: 09767357.8
(22) Date of filing: 28.05.2009
(51) Int. Cl.: A61M 25/10

(54) **NEW FUNCTIONAL BALLOON WITH BUILT IN LUBRICITY OR DRUG DELIVERY SYSTEM**
FUNKTIONSBALLON MIT EINGEBAUTEM SCHMIERUNGS- ODER MEDIKAMENTIERUNGSSYSTEM
NOUVEAU BALLONNET FONCTIONNEL AVEC SYSTÈME DE LUBRIFICATION OU D' ADMINISTRATION DE MÉDICAMENT INTÉGRÉS

(30) Priority: 18.06.2008 US 141606
(43) Date of publication of application: 15.06.2011
(73) Proprietor: Boston Scientific Scimed, Inc., Maple Grove, MN 55311 (US)
(72) Inventor: CHEN, John, Plymouth MN 55446 (US)
(74) Representative: Hauck Patent- und Rechtsanwälte
(86) International application number: PCT/US2009/045462
(87) International publication number: WO 2009/155045

(56) References cited:
- WO-A-01/49337
- US-A1- 2003 065 352
- US-A1- 2004 019 362
- US-A1- 2007 129 748
- US-B1- 6 355 013

## Description

### FIELD OF THE INVENTION

In some embodiments, this invention relates expandable medical balloons that have strips of lubricous material along their lengths and methods of making such medical balloons

### BACKGROUND OF THE INVENTION

Balloon catheters, having expandable balloon members located at the distal end ofthe balloon catheter, are employed in a variety of medical procedures including as dilatation devices for compressing atherosclerotic plaque which results in a narrowing of the arterial lining, and for delivery and expansion of prosthetic devices, such as stents, to a lesion site

One medical procedure where balloon catheters are employed is percutaneous transluminal coronary angioplasty, or balloon angioplasty, which is a minimally invasive, non-surgical means of treating peripheral and coronary arteries. This technique consists of inserting an uninflated balloon catheter into the affected artery. Dilation ofthe diseased segment of artery is accomplished by inflating the balloon which pushes the atherosclerotic lesion outward, thereby enlarging the arterial diameter. Angioplasty has become recognized as an efficient and effective method of opening stenoses in the vascular system.

In the most widely used form of angioplasty, a balloon catheter is guided through the vascular system until the balloon, which is carried at the distal end of a catheter shaft and which may carry an expandable stent, is positioned across the stenosis or lesion, i.e , vessel obstruction. The balloon is then inflated to apply pressure to the obstruction which is essentially remolded by pressing it against the inner wall of the vessel whereby the vessel is opened for improved flow. Due to the expansion of the balloon, the stent, which is situated on the balloon, is also expanded and released to aid in support and/or repair of the vessel wall.

A stent is typically crimped onto the balloon while the balloon is in a folded/wrapped configuration. Once the site of deployment has been reached, the balloon is inflated, thereby expanding the stent. The balloon is then deflated wherein it rewraps and is then removed from the body vessel.

To facilitate advancement of the catheter assembly through the body vessel, lubriciousness has been imparted to the catheter surfaces, including to the exterior surface of the expandable balloon member. Increasing the lubriciousness of the balloon surface can also facilitate withdrawal of the balloon from a deployed, expanded stent.

Balloons are typically made of polymeric materials including nylon, Selar(TM), polyetherpolyester block copolymers (e.g. Hytrel(TM) or Amitel(TM)), poly(amide-ether-ester) block copolymers such as Pebax(TM), polyethylene terephthalate, polytetrafluoroethylene, polyvinyl chloride, polyurethanes, polyetherurethanes, polyesterurethanes, polyurethane ureas, polyurethane siloxane block copolymers, polyethylene, polypropylene or other similar extrudable thermoplastic, polymeric materials, or composites thereof Such materials are typically inherently nonlubricious making it necessary to add some type of lubricious coating to the surface in order to advance the device through the blood vessel more easily.

Balloons will therefore typically have a lubricating portion and a non-lubricating portion to avoid what is referred to in the industry as the "watermelon seed" problem, wherein a balloon which is too lubricious shoots forward on inflation causing accidental slippage from the target site U.S. Pat. No. 5,503,631 to Onishi et al discloses a vasodilating catheter balloon whose body has a lubricating portion and a non-lubricating portion. The lubricious property of the balloon is created by applying a lubricious coating onto a non-lubricious substrate. Only the tapered portions on opposite ends of the balloon were treated.

US 2007/ 129748 A1 (closest prior art) discloses an expandable medical balloon having predetermined strategic arrangement of differentially lubricious surfaces. Lubricity by a lubricous coating is imparted to the surface in those areas between the folds of the balloon, such that the treated areas are not in contact with the crimped stent until deployment.

The present inventor has now found new methods for coating a balloon prior to formation of balloon which can achieve a selective lubricious coating for the use of a balloon catheter. The methods allow for flexibility in tailoring a desired balloon configuration for balloon catheters.

The art referred to and/or described above is not intended to constitute an admission that any patent, publication or other information referred to herein is "prior art" with respect to this invention, In addition, this section should not be construed to mean that a search has been made or that no other pertinent information as defined in 37 C.F.R. $156(a) exists.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

In at least one embodiment, the invention is directed to an expandable medical balloon. The expandable medical balloon has an inflated configuration and a deflated configuration and is formed from an extruded tubular segment, wherein the tubular segment comprises a tube of a first material. The tubular segment further includes two or more strips of a second material embedded in the tube by coextrusion with the first material. The two or more strips are outwardly exposed and extend longitudinally relative to the longitudinal axis of the tube and are more lubricious than the first material.

In some embodiments of the invention, the second material is a hydrophilic material. In some embodiments of the invention, the second material is a hydrophobic material.

In some embodiments of the invention, the second material comprises a water swellable material, wherein the water swellable material can contain one or more therapeutic agents.

In at least one embodiment, the invention is directed to an expandable medical balloon. The expandable medical balloon has an inflated configuration and a deflated configuration and is formed from an extruded tubular segment, wherein the tubular segment comprises a tube of a first material The tubular segment further includes two or more strips of a second material bonded to the tube, wherein the second material comprises a reactive group. The second material is coextruded with the first material. The expandable medical balloon further comprises a lubricous material, wherein the lubricous material is differentially bonded to the reactive groups of the second material through covalent bonds or ionic bonds and wherein the lubricous material is more lubricous than the first material. In some of the embodiment the reactive group is chosen from the group consisting of acids, alcohol, isocyanate, epoxy, amines and anhydrides

In some embodiments of the present invention, the expandable medical balloon is made by forming at tubular segment by coextruding a first material and a second material, wherein the first material is formed into a tube and the second material is formed into two or more outwardly exposed strips longitudinally embedded in the tube and wherein the first mater ial is less lubricious than the second material. Thereafter, the tubular segment is expanded to form a medical balloon.

In some embodiments of the present invention, the expandable medical balloon is made by forming at tubular segment by coextruding a first material and a second material, wherein the first material is formed into a tube and the second material is formed into two or more outwardly exposed strips longitudinally on the tube A lubricous material is thereafter applied to the tubular segment material. The lubricous material, which is more lubricous than the first material, differentially bonds to the second material through covalent bonds or ionic bonds The tubular segment is then washed, removing the lubricous material from the first material.

These and other embodiments which characterize the invention are pointed out with particularity in the claims annexed hereto and forming a part hereof. However, for further understanding of the invention, its advantages and objectives obtained by its use, reference can be made to the drawings which form a further part hereof and the accompanying descriptive matter, in which there is illustrated and described an embodiments ofthe invention.

### BRIEF DESCRIPTION OF THE SEVERAL VIEWS OF THE DRAWING(S)

A detailed description of the invention is hereafter described with specific reference being made to the drawings
FIG 1 is a side view of a catheter that includes a coated balloon of the present invention.
FIG 2 is a cross-section view of the balloon of FIG. 1 at line 2-2.
FIG. 3 is a perspective view of a preform segment from which a balloon of the present invention is made.
FIG. 4 is an end view ofthe preform segment of FIG. 3.
FIG 5 is a perspective view of a preform segment from which a balloon of the present invention is made.
FIG. 6 is an end view of the preform segment of FIG. 5.

### DETAILED DESCRIPTION OF THE INVENTION

While this invention may be embodied in many different forms, there are described in detail herein specific embodiments ofthe invention This description is an exemplification of the principles of the invention and is not intended to limit the invention to the particular embodiments illustrated.

For the purposes of this disclosure, like reference numerals in the figures shall refer to like features unless otherwise indicated.

Balloons of the invention are particularly suited to use in medical devices, for instance on balloon angioplasty catheters, in stent delivery systems, perfusion balloon devices, cutting balloon devices, cryoplasty devices, and the like. Typically they will be mounted on a catheter or probe device.

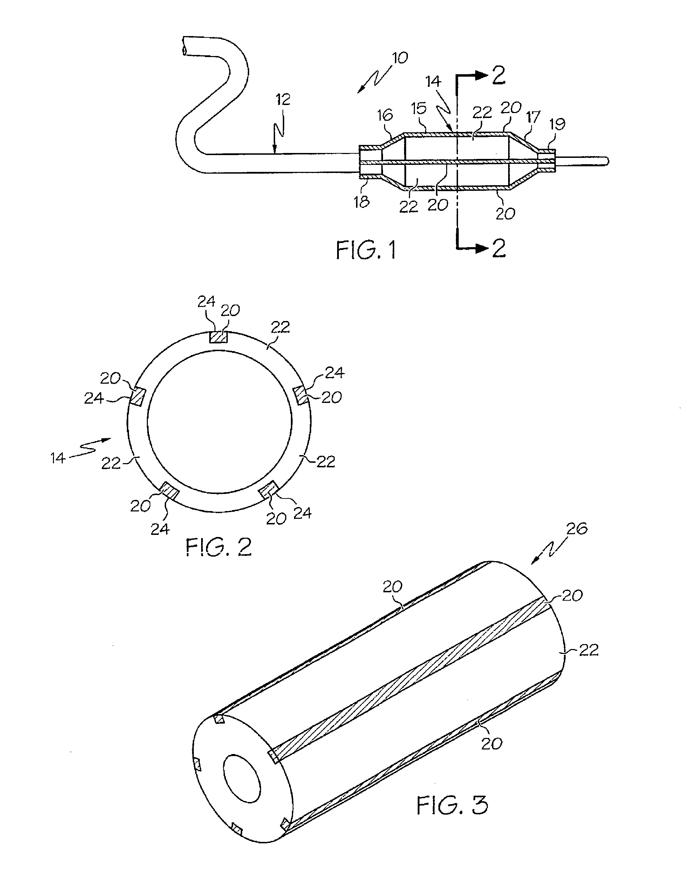

Referring to the drawing Figures 1-6, several aspects of the inventive processes are illustrated

A medical balloon catheter of the present invention, illustrated generally at 10 in FIG. 1, includes an inflatable balloon 14 mounted at the distal end of an elongated flexible shaft 12 Except as noted herein, catheter 10 is conventional in its construction, providing a lumen communicating with the interior of the balloon for inflation and deflation of the balloon and other optional features conventional in the dilatation catheter art The balloon 14 has an inflated configuration, as illustrated in FIG. 1, and is made up of a main body 15, a proximal cone 16, a distal cone 17, a proximal waist portion 18 and a distal waist portion 19.

FIG. 1 also illustrates strips 20 oflubricious material embedded in the primary balloon material 22. The strips 20 of lubricous material are shown to be linear and to extend the entire length of the balloon 14. The strips 20 can also be constructed to have lengths which vary The strips 20 can also spiral around the longitudinal axis of the balloon. A "strip" is a narrow, comparatively long piece of material In some embodiments, the width of the strip is uniform and in some embodiments, the width of the strip varies. The width and length of the strips can be tailored to meet the intended purpose of the balloon

FIG. 2 represents a cross-section of the medical balloon along lines 2-2 in FIG 1. As can be seen, the strips 20 are spaced around the circumference of the balloon 14. The strips 20 of lubricious material are embedded in the primary balloon material 22. In the embodiments ofthe present invention, the outwardly exposed portion 24 of the strips 20 is circumferentially flush with the outwardly exposed portions of the primary balloon material 22 In some embodiments, the outwardly exposed portion 24 of the strips 20 extend above the outwardly exposed portions of the primary balloon material 22.

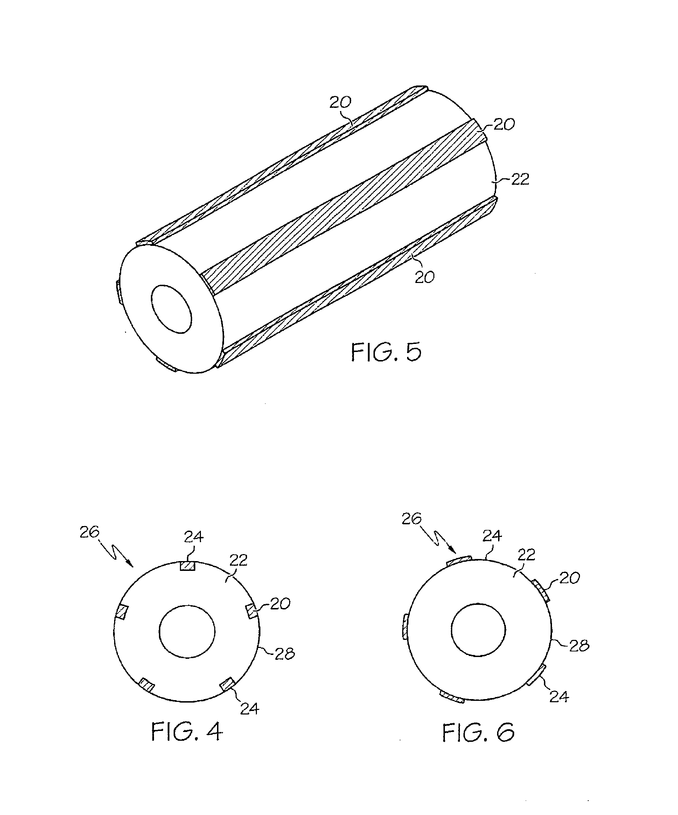

FIG. 3 represents a perspective view of a preform segment 26 according to the present invention FIG. 4 is an end view of the preform segment 26 from which the balloon 14 is blown The formation of a medical balloon from an extruded preform segment is well known.

As can be seen, prior to the blowing and forming the balloon 14 from the preform segment 26, the preform segment 26 has a greater thickness than that of the blown balloon 14. The internal pressure of the blowing process thins the primary material 22. Because of the expansion, the balloon 14 contains a thinner and larger layer of functional material on its surface than that ofthe preform segment 26 The functional material can be the primary balloon material 22 and/or the strip 20 material depending upon the desired use.

In the formation of the preform segment, an extrusion process is used The primary material 22 and the strips 20 of lubricous material are coextruded to form a tube A designated length of the tube is cut to form the preform segment 26. The preform segment is thereafter expanded in a conventional manner to form the balloon. The balloon is thereafter mounted onto a catheter.

As can be seen in FIGs 3 and 4, the coextruded strip 20 material is embedded in the primary material 22. In some embodiments, the outwardly exposed strip 20 material is circumferentially flush with the outwardly exposed primary material 28. In some embodiments, the outwardly exposed strip 20 material extends radially above or beyond the outwardly exposed primary material 28.

In one embodiment of the present invention, as shown in figure 5, the primary material 20 has a circular circumference and the strips 20 are coextruded onto and bonded to, via the extrusion process, the outer surface of the primary material 22

In some embodiments, the coextruded strip 20 material contains reactive groups The resulting preform segment from the coextrusion has reactive groups, in the configuration of strips, exposed on the surface ofthe primary material 22 The specific pattern of the strip of reactive groups can vary depending on the intended purpose of the balloon.

The formed preform segment 26 with the strips 20 of reactive groups is then coated with a lubricous coating The specific lubricous coating used is one that differentially bonds to the reactive groups. That is the coating bonds to the reactive groups and does not bond to the primary material 22. After the formed preform segment 26 is coated, the non-bonded lubricous coating material is washed away resulting in a preform segment 26 having strips 20 of a lubricous material which is more lubricous than the primary material 22

The lubricous coating which differentially bonds to the reactive groups is applied prior to blowing the balloon. The lubricous coating is applied to the entire preform segment 26 at a constant and consistent coating thickness. If the preform segment 26 is to be stretched, the coating can be applied before or after the stretching

The lubricous coating material and the material containing reactive groups chosen are compatible for covalent or ionic bonding to one another and, at the same time, the lubricous coating material that is chosen is not compatible for bonding with the primary material 22. The materials which include reactive groups include, but are not limited to, acids, alcohol, isocyanate, epoxy, amines and anhydrides

As an example, Pebax with stripes of functionalized polyolefin, such as anhydride modified, was coextruded into balloon tubing. The lubricous molecules of polyethyleneoxide (PEO) with hydroxyl or amine chain ends were made ready to react with anhydride or carboxylic groups on the surface to form partial lubricous balloon surface.

Another example is to coextrude a strip containing double bonds, then graft an initiator to the strip surface, and polymerization initiated by the grafted initiator to form poly(2-vinylpyridine) chains.

Another example is to graft Pcbax balloon surface with diisocyanate, then couple with hydroxyl chain end PEO or other water soluble materials. The pattern can be created with masking material to covet partial balloon surface in which the coating is not desired. After the chemical reactions, the masking material is removed to expose the original substrate. The masking material can be applied during extrusion or after balloon formation. During extrusion, for example, very thin polyolefin strips are coextruded on the top of Pebax balloon tubing Then the coextruded tubing is grafted with the functional group of isocyanate (through diisocyanate), and then coupled with water soluble molecules to form a partial lubricous coating on the tube. Other reactions can be found in U.S. Pat. No. 4,876,126 and Progress in Polymer Science 28 (2003) 209-259 & 32 (2007) 698-725.

The primary material 22 of the balloon can be made of thermoplastic polymeric materials including general classes such as thermoplastic elastomers, i.e. block copolymers; homopolymers, copolymers and terpolymers of ethylene; homopolymers, copolymers and terpolymers of propylene; ethylene alpha-olefins; polyesters; polyamides; polyurethanes; polycarbonates, vinyl copolymers; ionomer materials and so forth More specifically, materials such as nylon, Selar(TM), polyether-polyester block copolymers (i.e. Hytrel(TM)), Pebax(TM) (polyether block amide copolymers), Surlyn(TM), polyethylene terephthalate, polytetrafluoroethylene, polyvinyl chloride, polyetherurethanes, polyesteturethanes, polyurethane ureas, polyurethane siloxane block copolymers, silicone polycarbonate copolymers, ethylene vinyl acetate copolymers, actylonitrile-butadiene-styrene copolymers; polyphenylene sulfides; copolyesters or other similar extrudable thermoplastic, polymeric materials, or composites thereof. Can be utilized in the present invention. Thermosetting materials such as polyimides may also be utilized Other usable balloon primary material can be found in U.S. Pat. No. 5,135,516; U.S. Pat. No. 5,026,607; U.S. Pat. No. 5,304,121; U.S. Pat. No. 5,576,072; U.S. Pat. No. 5,503,631; U.S. Pat. No. 5,509,899; U.S. Pat. No. 5,693,034; U.S. Pat. No. 6,110,483; U.S. Pat. No. 5,702,756; U.S. Pat. No. 6,528,150; U.S. Pat. No. 6,673,053; and US Publication 2007/0267128. In some embodiments of the present invention, the balloon primary material can include polyether block amides, such as Pebax(TM) 7033 or 7233; polyesterblock ethers such as Amitel(TM) EM 740; polyethylene terephthalate; and nylon. The formation of catheter balloons made of block copolymer elastomers where the hard segments arc polyester or polyamide and the soft segments are polyether, is discussed in U.S. Pat. No. 5,556,383, issued Sep. 17, 1996, to Wang et al.

The lubricous coextruded strip material chosen is a material that adheres to the balloon primary material. In some embodiments, the lubricous strip material can be formed from any hydrophilic compound or any low friction hydrophobic coating which imparts lubricity to the balloon material. Examples of useful hydrophobic coatings include silicone lubricants or polymers and fluoropolymer coatings, which includes, but not limited to, PurSil 60. The method of the present invention for forming lubricity strips on the balloon itself is not dependent on the coating utilized. Of course, some coatings are more desirable than others.

There are many hydrophilic compounds that may be utilized in the present invention. The water soluble lubricants useful herein include Pebax MV1074, polyalkylene glycols, alkoxy polyalkylene glycols, homopolymers and copolymers of (meth)acrylic acid, copolymers of methylvinyl ether and maleic acid, poly(vinylpyrrolidone) homopolymers, copolymers of vinyl pyrrolidone, poly(N-alkylacrylamide), poly(vinyl alcohol), poly(ethyleneimine), polyamides, methyl cellulose, carboxymethylcellulose, polyvinylsulfonic acid, heparin, dcxtran, modified dextran, chondroitin sulphate and lecithin. The polymers are typically chain-structured, non-crosslinked and water soluble having a hydrophilic group such as -OH, -CONH2, -COOH, -NH2, -COO-, -SO3, -NR3<+> and so forth where R is alkyl or hydrogen. Further useful hydrophobic and hydrophilic materials are found in U.S. Publication No. 2007/0129748.

In some embodiments, the lubricous strip material includes one or more therapeutic agents. When a solution swellable lubricous strip material such as a water swellable material is used to create the functional strip surface, it can be loaded with therapeutic medicine by soaking it into the solution and drying out the solvent(s) afterwards. In such cases, the lubricous material can be a water absorbable material such as, but not limited to, polyacrylates based materials, polyactylamide, isobutylene maleic anhydride copolymers and poly(vinyl alcohol) based materials. The organic solvent can be the swelling agent and then the strip would not have to have lubricous function for drug carrying purposes.

Balloon formation may be carried out in any conventional manner with conventional extrusion and blowing techniques, but basically there are three major steps in the process which include extruding a tubular preform, blow molding the balloon and annealing the balloon. Depending on the balloon material employed, the preform segment may be axially stretched before it is blown. Techniques for balloon formation are discussed in U.S. Pat. No. 4,490,421, U.S. Pat. No. 5,348,538, U.S. Pat. No. 4,963,313, U.S. Pat. No. 5,306,246, U.S. Pat. No. 4,935,190, U.S. Pat. No. 5,714,110, U.S. Pat. No. 4,906,244, U.S. Pat. No. 5,328,468, U.S. Pat. No. 4,950,239, U.S. Pat. No. 5,500,180, U.S. Pat. No. 5,556,383, U.S. Pat. No. 6,146,356, U.S. Pat. No. 6,270,522, U.S. Pat. No. 5,344,400, U.S. Pat. No. 5,833,657, U.S. Pat. No. 5,250,069, U.S. Pat. No. 5,797,877, U.S. Pat. No. 5,270,086 and U.S. Pat. No. 5,304,340. Various known methods of altering the properties of a radially expanded balloon such as heat-setting, heat shrinking, and/or radiation crosslinking may also be employed in forming the balloon (see U.S. Pat. No. 5,403,340; EP 540858; and WO 98/03218. After the balloon is formed, the assembled balloon can be folded preferentially either with the lubricous surface on the outside and exposed or with the primary material surface on the outside and exposed based on the desired needs. Examples selective folding techniques are found in US Publication 200710129748. In some embodiments, a medical device can be mounted on the medical balloons of the present invention. Such medical devices include stents, grafts, stent-grafts, vena cava filters, expandable frameworks, and similar implantable medical devices. These medical devices are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, fallopian tubes, coronary vessels, secondary vessels, etc They may be self-expanding, expanded by an internal radial force, such as when mounted on a balloon, or a combination of self-expanding and balloon expandable (hybrid expandable).

In the embodiments that incorporate a therapeutic agent, the therapeutic agent may be a drug or other pharmaceutical product such as non-genetic agents, genetic agents, cellular material, etc Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate.

The above disclosure is intended to be illustrative and not exhaustive. This description will suggest many variations and alternatives to one of ordinary skill in this art. The various elements shown in the individual figures and described above may be combined or modified for combination as desired. All these alternatives and variations are intended to be included within the scope of the claims where the term "comprising" means "including, but not limited to".

Further, the particular features presented in the dependent claims can be combined with each other in other manners within the scope of the invention such that the invention should be recognized as also specifically directed to other embodiments having any other possible combination of the features of the dependent claims. For instance, for purposes of claim publication, any dependent claim which follows should be taken as alternatively written in a multiple dependent form from all prior claims which possess all antecedents referenced in such dependent claim if such multiple dependent format is an accepted format within the jurisdiction (e.g. each claim depending directly from claim 1 should be alternatively taken as depending from all previous claims). In jurisdictions where multiple dependent claim formats are restricted, the following dependent claims should each be also taken as alternatively written in each singly dependent claim format which creates a dependency from a prior antecedent-possessing claim other than the specific claim listed in such dependent claim below

This completes the description of the invention Those skilled in the art may recognize other equivalents to the specific embodiment described herein which equivalents are intended to be encompassed by the claims attached hereto.

### FURTHER EXPENDABLE MEDICAL BALLOONS AND METHODS ARE GIVEN BELOW:

1. An expandable medical balloon, the expandable medical balloon having an inflated configuration and a deflated configuration and being formed from an extruded tubular segment, wherein the tubular segment has a longitudinal axis and comprises a tube of a first material, wherein the tube has a length and is circumferentially closed, and two or more strips of a second material embedded in the tube, the second material being a coextrusion with the first material, wherein each of the two or more strips is outwardly exposed and extends longitudinally relative to the longitudinal axis of the tube and wherein the second material is more lubricious than the first material.
2. An expandable medical balloon according to paragraph 1 wherein each of the two or more strips extend the length of the tube.
3. An expandable medical balloon according to paragraph 1 wherein the tubular segment has a thickness and each of the two or more strips has a thickness, the thickness of the tubular segment being greater than the thickness of the two or more strips.
4. An expandable medical balloon according to paragraph 1, the first material of the tube of the tubular segment being outwardly exposed in an alternating fashion, in a circumferential direction, with the outward exposure of the second material.
5. An expandable medical balloon according to paragraph 1, wherein the second material is a hydrophilic material.
6. An expandable medical balloon according to paragraph 1, wherein the second material is a hydrophobic material.
7. An expandable medical balloon according to paragraph 1, the second material comprising a therapeutic agent.
8. An expandable medical balloon according to paragraph 4, wherein the outwardly exposed first material of the tubular segment is circumferentially flush with the outwardly exposed second material.
9. An expandable medical balloon of paragraph 1, wherein, in a circumferential direction around the expandable medical balloon, outwardly exposed first material alternates with outwardly exposed second material.
10. An expandable medical balloon of paragraph 1, wherein, when the expandable medical balloon is folded and has an outwardly exposed surface, the second material comprises a majority of the outwardly exposed surface.
11. An expandable medical balloon of paragraph 1, wherein, when the expandable medical balloon is folded and has an outwardly exposed surface, the first material comprises a majority of the outwardly exposed surface.
12. An expandable medical balloon of paragraph 1, wherein the second material comprises a water swellable material.
13. An expandable medical balloon of paragraph 1, wherein the first material is a polyamide/polyether polyester.
14. An expandable medical balloon, the expandable medical balloon having an inflated configuration and a deflated configuration and being formed from an extruded tubular segment, wherein the tubular segment has a longitudinal axis and comprises a tube of a first material, wherein the tube has a length and is circumferentially closed, and two or more strips of a second material bonded to the tube, wherein the second material comprises a reactive group, the second material being a coextrusion with the first material, wherein each of the two or more strips is outwardly exposed and extends longitudinally relative to the longitudinal axis of the tube, the expandable medical balloon further comprising a lubricous material, wherein the lubricous material is differentially bonded to the reactive groups of the second material, the lubricous material being more lubricous that the first material.
15. An expandable medical balloon according to paragraph 14 wherein each of the two or more strips extend the length of the tube.
16. An expandable medical balloon according to paragraph 14 wherein the tubular segment has a thickness and each of the two or more strips has a thickness, the thickness of the tubular segment being greater than the thickness of the two or more strips.
17. An expandable medical balloon according to paragraph 14 the first material of the tube of the tubular segment being outwardly exposed in an alternating fashion, in a circumferential direction, with the outward exposure of the second material.
18. An expandable medical balloon according to paragraph 14, wherein the lubricous material is a hydrophilic material.
19. An expandable medical balloon according to paragraph 14, wherein the lubricous material is a hydrophobic material.
20. An expandable medical balloon according to paragraph 14, the lubricous material comprising a therapeutic agent.
21. An expandable medical balloon according to paragraph 14, wherein the reactive group is chosen from the group consisting of acids, alcohols, amines, isocyanates, epoxies, anhydrides and alkenes.
22. An expandable medical balloon of paragraph 14, wherein, when the expandable medical balloon is folded and has an outwardly exposed surface, the lubricous material comprises a majority of the outwardly exposed surface.
23. A method of making an expandable medical balloon, comprising the steps:
   forming at tubular segment by coextruding a first material and a second material, wherein the first material is formed into a tube and the second material is formed into two or more outwardly exposed strips longitudinally embedded in the tube, the first material being less lubricious than the second material; and
   expanding the tubular segment to form a medical balloon.
24. A method of making an expandable medical balloon, comprising the steps:
   forming at tubular segment by coextruding a first material and a second material, wherein the first material is formed into a tube and the second material is formed into two or more outwardly exposed strips longitudinally on the tube; and
   applying a lubricous material to the tubular segment, wherein the lubricous material differentially bonds to the second material and is more lubricous than the first material.
25. The method of paragraph 24, further comprising the step of washing the tubular segment, such that the lubricous material is removed from the first material and left on the second material through covalent bonds or ionic bonds.

## Claims

1. An expandable medical balloon, the expandable medical balloon (14) having an inflated configuration and a deflated configuration and being formed from an extruded tubular segment, wherein the tubular segment has a longitudinal axis and comprises a tube of a first material (22), wherein the tube has a length and is circumferentially closed, and two or more strips (20) of a second material embedded in the tube, the second material being a coextrusion with the first material, wherein each of the two or more strips (20) is outwardly exposed and extends longitudinally relative to the longitudinal axis of the tube and wherein the second material is more lubricious than the first material and the first material (22) of the tube of the tubular segment being outwardly exposed in an alternating fashion, in a circumferential direction, with the outward exposure of the second material **characterized in that** the outwardly exposed first material (22) of the tubular segment is circumferentially flush with the outwardly exposed second material.

2. An expandable medical balloon (14) according to claim 1 wherein each of the two or more strips (20) extend the length of the tube.

3. An expandable medical balloon (14) according to claim 1 wherein the tubular segment has a thickness and each of the two or more strips (20) has a thickness, the thickness of the tubular segment being greater than the thickness of the two or more strips (20).

4. An expandable medical balloon (14) according to claim 1, wherein the second material is a hydrophilic material.

5. An expandable medical balloon (14) according to claim 1, wherein the second material is a hydrophobic material.

6. An expandable medical balloon (14) according to claim 1, the second material comprising a therapeutic agent.

7. An expandable medical balloon (14) of claim 1, wherein, in a circumferential direction around the expandable medical balloon (14), outwardly exposed first material (22) alternates with outwardly exposed second material.

8. An expandable medical balloon (14) of claim 1, wherein, when the expandable medical balloon (14) is folded and has an outwardly exposed surface, the second material comprises a majority of the outwardly exposed surface.

9. An expandable medical balloon (14) of claim 1, wherein, when the expandable medical balloon (14) is folded and has an outwardly exposed surface, the first material (22) comprises a majority of the outwardly exposed surface.

10. An expandable medical balloon (14) of claim 1, wherein the second material comprises a water swellable material.

11. An expandable medical balloon (14) of claim 1, wherein the first material is a polyamide/polyether polyester.

## Patentansprüche

1. Aufblähbarer medizinischer Ballon, wobei der aufblähbare medizinische Ballon (14) eine aufgeblähte Konfiguration und eine entleerte Konfiguration aufweist und aus einem extrudierten röhrenförmigen Segment erzeugt wurde, wobei das röhrenförmige Segment eine Längsachse hat und eine Röhre aus einem ersten Material (22) umfasst, wobei die Röhre umlaufend geschlossen ist und eine Länge sowie zwei oder mehr in der Röhre eingebettete Streifen (20) aus einem zweiten Material aufweist, wobei das zweite Material eine Koextrusion mit dem ersten Material ist, wobei jeder der zwei oder mehr Streifen (20) nach außen hin frei liegt und sich in Längsrichtung in Bezug auf die Längsachse der Röhre erstreckt und wobei das zweite Material gleitfähiger als das erste Material ist und das erste Material (22) der Röhre des röhrenförmigen Segments in einer Weise nach außen hin frei liegt, die mit dem Freiliegen des zweiten Materials in einer Umfangsrichtung alterniert, **dadurch gekennzeichnet, dass** das nach außen hin frei liegende erste Material (22) des röhrenförmigen Segments in Umfangsrichtung bündig mit dem nach außen hin frei liegenden zweiten Material ist.

2. Aufblähbarer medizinischer Ballon (14) gemäß Anspruch 1, wobei jeder der zwei oder mehr Streifen (20) sich über die Länge der Röhre erstreckt.

3. Aufblähbarer medizinischer Ballon (14) gemäß Anspruch 1, wobei das röhrenförmige Segment eine Dicke hat und jeder der zwei oder mehr Streifen (20) eine Dicke hat, wobei die Dicke des röhrenförmigen Segments größer ist als die Dicke der zwei oder mehr Streifen (20).

4. Aufblähbarer medizinischer Ballon (14) gemäß Anspruch 1, wobei das zweite Material ein hydrophiles Material ist.

5. Aufblähbarer medizinischer Ballon (14) gemäß Anspruch 1, wobei das zweite Material ein hydrophobes Material ist.

6. Aufblähbarer medizinischer Ballon (14) gemäß Anspruch 1, wobei das zweite Material ein therapeutisches Mittel umfasst.

7. Aufblähbarer medizinischer Ballon (14) gemäß Anspruch 1, wobei in einer Umfangsrichtung um den ausdehnbaren medizinischen Ballon (14) herum nach außen hin frei liegendes erstes Material (22) sich mit nach außen hin frei liegendem zweiten Material abwechselt.

8. Aufblähbarer medizinischer Ballon (14) gemäß Anspruch 1, wobei wenn der ausdehnbare medizinische Ballon (14) zusammengefaltet ist und eine nach außen hin frei liegenden Oberfläche hat, das zweite Material eine Mehrheit der nach außen hin frei liegenden Oberfläche ausmacht.

9. Aufblähbarer medizinischer Ballon (14) gemäß Anspruch 1, wobei wenn der ausdehnbare medizinische Ballon (14) zusammengefaltet ist und eine nach außen hin frei liegenden Oberfläche hat, das erste Material eine Mehrheit der nach außen hin frei liegenden Oberfläche ausmacht.

10. Aufblähbarer medizinischer Ballon (14) gemäß Anspruch 1, wobei das zweite Material ein wasserquellbares Material umfasst.

11. Aufblähbarer medizinischer Ballon (14) gemäß Anspruch 1, wobei das erste Material ein Polyamid/Polyether-Polyester ist.

## Revendications

1. Ballonnet médical expansible, ledit ballonnet médical expansible (14) ayant une configuration gonflée et une configuration dégonflée et étant formé d'un segment tubulaire extrudé, chez ledit ballonnet le segment tubulaire a un axe longitudinal et comporte un tube d'un premier matériau (22), le tube a une longueur et est circonférentiellement fermé et a deux ou plus bandes (20) d'un deuxième matériau encastrées dans le tube, le deuxième matériau étant une co-extrusion avec le premier matériau, chacune des deux ou plus bandes (20) étant exposée vers l'extérieur et s'étendant longitudinalement par rapport à l'axe longitudinal du tube, le deuxième matériau étant plus lubrique que le premier matériau, le premier matériau (22) du tube du segment tubulaire étant exposé vers l'extérieur dans le sens circonférentiel de manière alternante avec l'exposition vers l'extérieur du deuxième matériau, **caractérisé en ce que** le premier matériau (22) exposé vers l'extérieur du segment tubulaire est circonférentiellement affleurant avec le deuxième matériau exposé vers l'extérieur.

2. Ballonnet médical expansible (14) selon la revendication 1, chez lequel chacune des deux ou plus bandes (20) s'étend à travers la longueur du tube.

3. Ballonnet médical expansible (14) selon la revendication 1, chez lequel le segment tubulaire a une épaisseur et chacune des deux ou plus bandes (20) a une épaisseur, l'épaisseur du segment tubulaire étant plus grande que l'épaisseur des deux ou plus bandes (20).

4. Ballonnet médical expansible (14) selon la revendication 1, chez lequel le deuxième matériau est un matériau hydrophile.

5. Ballonnet médical expansible (14) selon la revendication 1, chez lequel le deuxième matériau est un matériau hydrophobe.

6. Ballonnet médical expansible (14) selon la revendication 1, chez lequel le deuxième matériau comporte un agent thérapeutique.

7. Ballonnet médical expansible (14) selon la revendication 1, chez lequel, dans un sens circonférentiel autour du ballonnet médical expansible (14), du premier matériau (22) exposé vers l'extérieur alterne avec du deuxième matériau exposé vers l'extérieur.

8. Ballonnet médical expansible (14) selon la revendication 1, chez lequel quand le ballonnet médical expansible (14) est plié et a une surface exposée vers l'extérieur, le deuxième matériau comporte une majorité de la surface exposée vers l'extérieur.

9. Ballonnet médical expansible (14) selon la revendication 1, chez lequel quand le ballonnet médical expansible (14) est plié et a une surface exposée vers l'extérieur, le premier matériau (22) comporte une majorité de la surface exposée vers l'extérieur.

10. Ballonnet médical expansible (14) selon la revendication 1, chez lequel le deuxième matériau comporte un matériau gonflable dans l'eau.

11. Ballonnet médical expansible (14) selon la revendication 1, chez lequel le premier matériau est un polyester de polyamide/polyéther.
